(19) Europäisches Patentamt
European Patent Office
Office européen des brevets

(11) **EP 2 052 784 A1**

(12) **EUROPEAN PATENT APPLICATION**

(43) Date of publication:
**29.04.2009 Bulletin 2009/18**

(51) Int Cl.:
***B03C 5/02*** *(2006.01)*

(21) Application number: **07119255.3**

(22) Date of filing: **25.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **22.10.2007 EP 07118964**

(71) Applicant: **Koninklijke Philips Electronics N.V.
5621 BA Eindhoven (NL)**

(72) Inventor: **The designation of the inventor has not yet been filed**

(74) Representative: **Rüber, Bernhard Jakob
Philips Intellectual Property & Standards GmbH
Postfach 50 04 42
52088 Aachen (DE)**

(54) **Barrier layer over electrodes of electrically active loc devices**

(57)    A barrier layer, e.g. an electrically permeable layer such as a hydrogel layer (30) is provided on electrodes (50) of a dielectrophoretic device for the purpose of preserving the viability of cells and in particular preventing electric field-induced cell damage and lysis. The barrier layer provides a physical barrier that keeps the cells at a certain distance from the surface of the electrodes, where the electric field has the highest intensity. In contrast to an insulating layer, the hydrogel layer is essentially permeable to the electric field. The electric field on the surface of the layer is therefore still sufficient for the dielectrophoretic manipulation of the cells such as trapping the cells in a field cage. The device can be integrated into Lab-on-chip (LOC) microsystems.

FIG 1

EP 2 052 784 A1

**Description**

This invention relates to devices for applying an electric field to particles in a fluid, to methods of using such devices for manipulating particles, and to methods of manufacturing such devices. More particularly, it relates to devices or microsystems using dielectrophoresis to move particles.

Lab-on-a-chip (LOC) systems show great promise for a variety of medical, life-science, forensic and environmental applications. Their advantages include ease of use and low fabrication costs (ultimately leading to disposable chips), low fluid volumes and reagent consumption, large integration of functionalities, high-throughput analysis via massive parallelization and increased process control due to the faster response of the system. Initially, much emphasis was placed on developing LOC systems for genetic (DNA) analysis. As DNA microchips are now well established, the research focus is rapidly shifting to the analysis of proteins and even more complex biological systems, such as living cells.

One of the most promising methods to separate and manipulate cells in microsystems is dielectrophoresis (DEP), i.e., the movement of dielectric particles in a nonuniform (usually AC) electric field. Unlike electrophoresis, DEP relies on field-induced polarization effects and is independent of the net charge of the particle. The DEP force depends on the electrical properties of the particle and of the surrounding medium, on the size (and shape) of the particle and on the spatial distribution and frequency of the applied field. Depending on these factors, the particle can be attracted to either high-field (positive DEP) or low-field (negative DEP) regions. By using proper electrode configurations and multiphase fields, DEP can be used to levitate particles, trap them in a field cage in the form of a trough or peak in the field, rotate them (electrorotation) or transport them over relatively long distances (traveling wave DEP).

An object of the invention is to provide improved devices for applying an electric field to particles in a fluid, to improved methods of using such devices for manipulating particles, and to improved methods of manufacturing such devices. More particularly, it relates to improved devices or microsystems using dielectrophoresis to move particles.

According to a first aspect, the invention provides:

A device for applying an electric field to particles in a fluid, the device having electrodes arranged to generate the electric field, for use with drive circuitry to drive the electrodes at selectable AC frequencies, the device also having a barrier layer to keep the particles in the fluid away from the areas of higher electric field intensity. The drive circuitry can be part of the device or separate from the device. The barrier layer is e.g. an electrically permeable layer. The dielectric properties of the barrier are such that it does not alter the field distribution significantly, i.e. is permeable to the field. High field intensity can occur at the electrode surface, but also in other locations, e.g. at channel restrictions, close to dielectric barriers etc...

Among a number of advantages of such a barrier layer is reduced exposure of the particles to high electric fields at the surface of the electrode. Such high electric fields can alter or damage the particles. It is known, for instance, that cells can be subject to lysis or electroporation by high electric fields.

According to this aspect of the present invention AC fields are used with (living) cells. The barrier layer thickness of the layer is selected to provide a degree of field shielding. The physical thickness of the barrier layer means that the cells cannot get that close to an electrode and hence experience a lower filed strength. Embodiments within this aspect of the invention can have any additional features, and some such additional features are set out in dependent claims, and some are set out in the examples in the detailed description.

One such additional feature is the device being arranged for dielectrophoresis.

One such additional feature is that the barrier layer is made of a material that has a sufficient thickness so that the field strength in the fluid at the interface between the fluid and the barrier layer is reduced so that the risk of lysis of a cell is also reduced while still allowing a sufficient electric field to achieve DEP, e.g. negative or positive DEP, or to achieve that a change from negative to positive DEP (or vice versa) results in an observable change of position of the cell.

One such additional feature is the barrier layer being permeable to the electric field. By making the barrier layer permeable, the field strength in the fluid is reduced much less than a reduction seen if the barrier layer is a non permeable material such as a perfect insulator. The dielectric constant and conductivity are preferably as close as possible to or the same as those of the fluid.

Another such additional feature is the barrier layer being formed of a hydrogel material. A useful feature of hydrogels is that they are optically transparent. Accordingly, for cell analysis the barrier layer will not affect the optical pathway and hence will allow detection of movement of the cells.

Another such additional feature is the barrier layer being of such a thickness, and the drive circuitry being arranged such that the field strength in the fluid adjacent to the barrier layer is sufficiently low to avoid cell lysis.

Another such additional feature is the barrier being thicker at parts of the electrode surface subject to higher electrical field strength. For example only some areas need to be covered with the barrier layer, and the rest can be left bare. The feature is useful because a high electric field can often occur at electrode edges or corners. It can be sufficient to cover these areas to avoid damaging the particles. The perturbation of the field due to the layer can be further minimized if only the edges are covered, compared to a uniform coating. In an aspect of the present invention a method of manufacturing such an intermitten coating is provided, e.g. these materials can be patterned by suitable methods such as photolithography or with other means.

Another such additional feature is the device being arranged to detect a crossover frequency for at least one of the particles, the drive circuitry being arranged to alter the AC frequency, so that the net force on the particle changes direction between the directions of increasing field strength and decreasing field strength and the device being arranged to detect such a change. This is useful in for example determining a state of the cell. For example the membrane porousity for instance or permeability of a cell to fluids or particles.

Another such feature is the electrodes and the drive circuitry being arranged so as to provide an electric field in the fluid in the form of a trough or peak, to retain the particles which have a net force in a direction of decreasing or increasing field strength respectively.

Another such feature is the device being an integrated micro fluidic device having integrated fluid handling components.

Another aspect of the invention provides a method of manipulating cells in a fluid using a dielectrophoretic device, the device having electrodes arranged to generate the electric field for use with circuitry to drive the electrodes at selectable AC frequencies, the device having a barrier layer on a surface of the electrodes facing the fluid, to keep the cells in the fluid away from the surface of the electrodes, the method having the steps of causing cells to enter the device, and controlling the AC frequency to cause the cells to move in a desired direction in the fluid.

An additional feature is the step of measuring a frequency at which crossover occurs. the crossover frequency, e.g. the frequency at which the DEP response changes from negative (repulsive) to positive (attractive) or vice versa, is highly influenced by the condition of the cell membrane. For example, just prior to rupture of a cell membrane it has been found that the DEP crossover frequency of that cell increases significantly. The cross-over frequency or a change therein may be used for a variety of tasks, e.g. related to diagnosis.

The cells can be e.g. living cells having cell membranes, and the method can further comprise altering the conditions so that the permeability of the cell membranes is altered and recording the crossover frequency again. This can be useful in diagnosis, therapy or fundamental medical research. For example, the altered conditions may include addition of a chemical agent that permeabilizes the cells, or changing a temperature.

Another aspect provides a method of manufacturing a dielectrophoresis device for applying an electric field to particles in a fluid, the method having the steps of forming electrodes arranged to generate the electric field, forming drive circuitry to drive the electrodes at selectable AC frequencies, and forming a barrier layer on a surface of the electrodes facing the fluid, to keep the particles in the fluid away from the surface of the electrodes.

Any of the additional features can be combined together and combined with any of the aspects. Other advantages will be apparent to those skilled in the art, especially over other prior art. Numerous variations and modifications can be made without departing from the claims of the present invention. Therefore, it should be clearly understood that the form of the present invention is illustrative only and is not intended to limit the scope of the present invention.

How the present invention may be put into effect will now be described by way of example with reference to the appended drawings, in which:

| | |
|---|---|
| FIG. 1 | shows a plan view of a quadrupolar dielectrophoretic trap, showing electric potential represented by a color map, while the contour lines are the lines where the electric field is of equal strength. A nearly circular field trap is visible at the center of the device. |
| FIGs. 2a, 2b and 2c | show plan view of a trap in use for measurements of DEP cross-over frequency. At low frequencies the cell is trapped in the center of the device by negative DEP (fig 2a). The frequency is then increased until positive DEP occurs (fig 2b). By approaching the electrode the cell is subjected to a high electric field, which can disrupt the cell membrane and cause lysis (fig 2c). |
| FIG. 3 | shows the electric field generated by quadrupolar electrodes in water. The regions where the electric field exceeds 1/3 of the maximum value in the device are indicated by darker shading. |
| FIG 4 | shows a graph showing z-dependence of the electric field at the location indicated by the cross in fig 3. |
| FIG. 5 | shows an embodiment of the invention showing a barrier layer in the form of a hydrogel layer deposited on top of microelectrodes in a DEP device to prevent cell lysis. The electric properties of the hydrogel are similar to those of the medium in which the cells are suspended. The layer is therefore permeable to the electric field and DEP trapping can still occur effectively. |
| FIGs. 6 and 7 | show the quadrupolar traps used for the lysis experiment. The device in Fig 6 has bare electrodes, whereas the one in Fig 7 has been coated with a hydrogel layer. DEP trapping of cells was observed in both devices. In contrast, the occurrence of cell lysis was drastically reduced by the hydrogel layer. |
| FIG. 8 | shows a graph showing levitation height of 20 $\mu$m polystyrene beads in water. The hydrogel |

layer only slightly reduces the DEP force experienced by the beads.

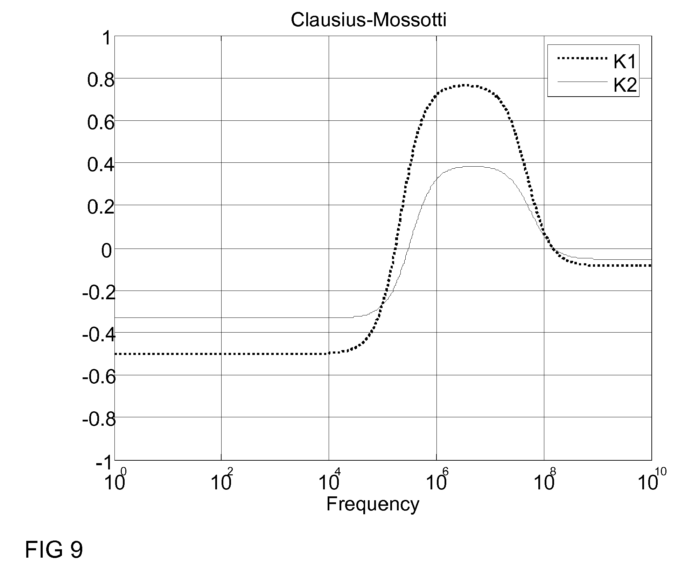

FIG 9 shows a graph which illustrates differing crossover frequencies to be used with embodiments of the invention.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may do so. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

By way of introduction to the embodiments, some problems or disadvantages addressed by some of the embodiments will be discussed. DEP has been applied to manipulate and separate a variety of cells including bacteria, yeast, and mammalian cells in microsystems. In particular, DEP has been used to separate cancer cells from blood, isolate CD34+ stem cells from blood, bacteria from blood and to separate various cell sub-populations of blood.

Known DEP methods do not say much about the nature of the cells, e.g. important properties of the cells. For example, traditional methods for determining the permeability state of the cell membrane usually rely upon evaluating the uptake of colorimetric or fluorescent dyes by the cells. However, these methods cannot be used for real-time monitoring of variations in the membrane integrity. Also, the addition of a dye (which stays in the cell after transfection) may not be desirable. A recent new method of measuring membrane integrity in real-time was proposed by Huang and Rubinski [Huang, Y. and Rubinsky, B. "Microfabricated electroporation chip for single cell membrane permeabilization." Sensors and Actuators A 89 (2001) 242-249; Huang, Y. et al. "Instantaneous, quantitative single-cell viability assessment by electrical evaluation of cell membrane integrity with micro fabricated devices." Sensors and Actuators A 105 (2003) 31-39]. Their approach is based on direct measurement of the conductivity of the cell membrane. In this device, a single cell is positioned such that it blocks a micro hole in a non-conducting membrane, and the conductivity between the opposite sides of the membrane is subsequently measured.

US 2007/010367 discloses an array of DEP cages in the form of an array of sensors. The content of this document is incorporated by reference in its entirety. This particularly relates to the formation of arrays of electrodes for DEP.

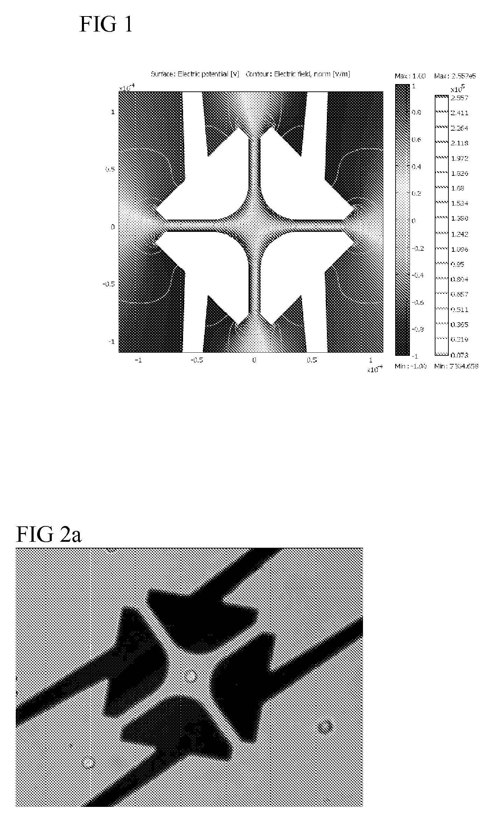

An example of a field trap is shown in figure 1. This shows four electrodes arranged around the fluid containing the particles. A problem with applying electric fields (DC or AC) is that they can induce profound changes in cells by perturbing the trans-membrane potential. The application of an external potential leads to the formation of transient pores in the membrane and in some cases to a permanent disruption of the cell membrane (lysis). These effects are exploited for example to introduce foreign macromolecules through the cell membrane (transfection), extract intracellular material, or perform cell fusion. In most applications, however, it is essential to be able to preserve the viability of the cells under investigation and therefore prevent or reduce field-induced damages. Dielectrophoretic devices are therefore usually operated in the regime of negative DEP, in which the cells are attracted to low field regions. This provides a very gentle means for transporting and focusing cells and for holding them in field traps.

In many cases there is a spread in the dielectric properties of the cells, which can result in some cells experiencing positive DEP while other experience negative DEP. The electric field can then result in lysing of specific fractions of the cell population.

In addition, when the field is modulated cells in the proximity of the electrodes can be damaged by the high electric field before they have the time to move to the low field regions. Cells can also be dragged towards high-field regions by fluid flow, which is a common means of transporting suspension cells in microfluidic devices and can result in forces much larger than the DEP force (in the order of 0.1-1nN for cells).

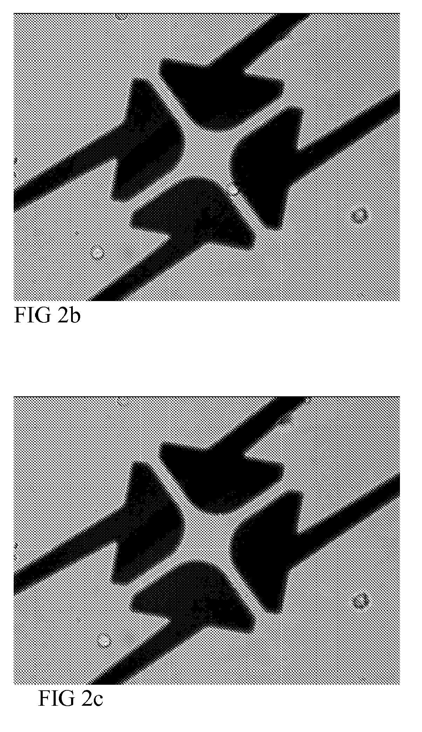

There is a particular risk of cell damage in cases where it is necessary to operate dielectrophoretic devices in the positive DEP regime, in which the cells are attracted towards the high field regions. An example is the measurement of the DEP cross-over frequency of cells, i.e., the frequency of the applied voltage at which the transition from negative to positive DEP occurs (Fig. 2). This frequency is an important parameter for cell analysis and provides valuable information on the characteristics of the cell, e.g., its viability degree. The cross over-frequency is generally measured as follows: First, a low frequency (e.g. 10 kHz) signal is applied to the electrodes to trap one or more cells in a field cage by negative DEP, as shown by fig 2a. The frequency of the applied signals is then increased until the cell(s) leave the center of the trap and start moving towards the electrodes as shown in fig 2b. The frequency is then lowered again to re-establish negative DEP, shown in fig 2c. Although positive DEP is induced only for a short time, there is a high risk of the cells being drawn too close to the electrodes and being damaged by the high electric field.

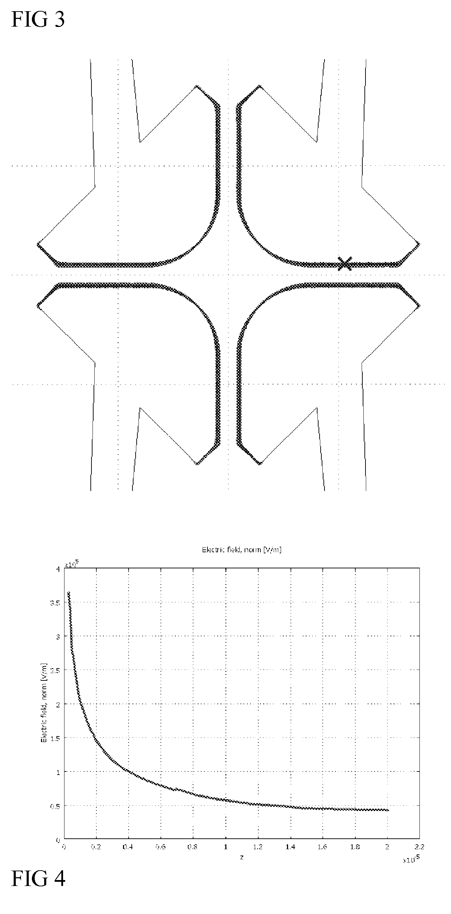

The electric field is especially high at the edges of the electrodes. Figure 3 shows the electric field generated by a quadrupolar trap in water with a voltage difference of 2V between adjacent electrodes. As can be seen, electric field values exceeding 1/3 of the maximum value in the device only occur in a very small region at the electrodes edges, indicated in darker shaded areas in the figure.

Fig. 4 shows a line scan of the electric field in the z-direction at the location indicated by the cross on the quadrupole. As expected, the electric field is maximum at the electrode surface and decreases rapidly, e.g. exponentially in the vertical direction. The present invention relies on the fact that electric field-induced cell damage and lysis can be reduced significantly by confining the cells outside of the region of high electric field (indicated by darker areas at the edges of the electrodes in figure 3). However, this raises a conflict - if the electric field is weakened too much DEP may be affected negatively, e.g. trapping by negative DEP may be impaired or there will be little or no change in the position of a cell undergoing a cross-over from negative to positive DEP.

In order to resolve this conflict while preserving the viability of cells and preventing electric field-induced damage, the electrodes are covered with a barrier layer. This layer can provide a physical barrier that keeps the cells at a certain distance from the surface of the electrodes, where the electric field has the highest intensity while still allowing the cells to be influenced by the field.

Other materials for the barrier layer are included within the scope of the present invention, providing that they do not screen the AC field significantly, For example: porous membranes (the pores will be filled by the medium), or any material with the same conductivity/dielectric constant then the medium. The barrier layer is preferably optically transparent, e.g. for imaging with a camera. In such a case hydrogels are preferred. Scattering by the porous membrane can be reduced by making the membrane thin and/or using a low-index of refraction materials to match the index of refraction of the aqueous fluid

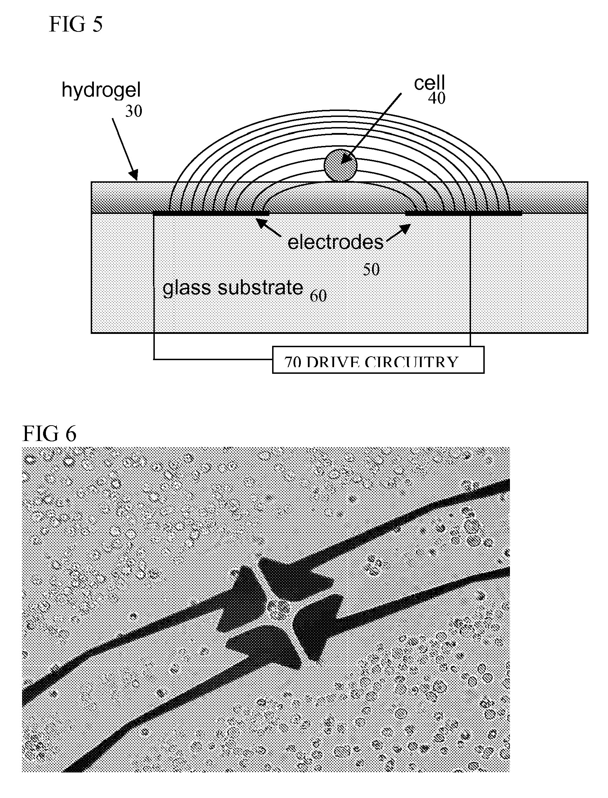

Figure 5 shows a cross section view of some elements of an embodiment. Electrodes 50 are formed on a glass substrate 60. An electrically permeable layer, e.g. a hydrogel barrier layer 30 covers the electrodes and keeps a cell 40 at a suitable distance away from the electrodes. Shown schematically in this figure is drive circuitry 70 for generating an AC field by applying AC currents of different phases to the electrodes in accordance with conventional DEP systems, e.g. driving circuits able to generate variable frequencies for nDEP or pDEP. Embodiments of the present invention can include such a variable frequency and phase AC field generator. The connections to the electrodes from the variable frequency and phase AC field generator are also not shown. Such parts that are not shown in detail can be implemented using conventional hardware or circuitry as would be apparent to those skilled in the art, and so need not be described in more detail. They can be integrated on the same chip or located elsewhere.

Also not shown in this figure is a cell presence and/or a cell movement detection system, e.g. a video camera system and image analysis system able to detect the position of cells and optionally their movement. The cell presence or movement sensing device may be an optical sensing device such as a photodiode, PIN diode, phototransistor, photoresistor, etc. A light source (which includes ambient light) may pass through the cage and strike the optical sensing device. In such a case, the optical sensing device is adapted to measure the reduction in received light due to the 'shadow' of a cell or cells when this or these are present in the cage. Although an optical sensor is a preferred embodiment, other sensors can be used. For example magnetic field sensors may be used when the cell or cells have a magnetic property, e.g. they are tagged with magnetic beads. Other suitable sensors can be sensors that detect the heat output of viable cells in the cage such as microcalorimetric measuring devices. Electrical activity of cells may be detected by means of an adapted Field Effect Transistor, e.g. by exposing a gate of an FET below the trap position such that when a cell rests on the exposed gate, the FET gives an output.

The barrier layer can be formed by a hydrogel, e.g. a network of (natural or synthetic) polymer chains that are water-insoluble, though other materials can be envisaged which can provide suitable permeability similar to that of the fluid. Other examples of materials that may be used are porous membranes such as made from cellulose acetate, cellulose nitrate, polyamide, poly(meth)acrylates, polyacrylamides etc.... A continuous layer is included within the scope of the present invention, but it should have preferably the same electric properties as the medium.

Use of hydrogels or other materials as barrier layers on electrodes, and how to manufacture them is shown in US6838053. In this document they are used for the different purpose of protecting the electrodes from electrochemical damage. Hydrogels are super-absorbent and can contain over 90% water. In contrast to an insulating layer such as SU8, the hydrogel layer is essentially permeable to the electric field due to its significant water content. The electric field at the surface of the hydrogel is therefore disturbed only marginally by the presence of the layer. The risk of lysis is reduced due to the fact that the hydrogel barrier creates a separation between the electrodes and the cell. In particular, the field at the surface of the hydrogel is still sufficient to manipulate and control the cells via DEP (e.g., trapping the cells in a field cage). Hydrogels are also known for being bio-compatible material and are for example used as scaffolds in tissue engineering.

Materials for hydrogels can be based on:

i) Anionic monomers: e.g., (meth)acrylic acid, p-styrene sulfonic acid, itaconic acid ($CH_2C(COOH)CH_2COOH$), crotonic acid ($CH_3CHCHCOOH$);
ii) Cationic monomers: e.g., vinyl pyridine, aminoethyl (meth)acrylates,;
iii) Neutral monomers: e.g., hydroxyethyl (meth)acrylate, vinyl acetate, and acrylamide. Neutral hydrogels are often preferred. However, incorporation of ions in the hydrogel will result in a charged gel but this need not have a significant effect with an AC field. Moreover, a charged gel might react to changes in pH of the fluid by expanding or shrinking.

Prior to deposition of the hydrogel layer, the substrate should be cleaned thoroughly (e.g., with UV ozone or plasma treatment) and coated with an adhesion promoter such as 3-Methacryloxypropyltrimethoxysilane (A174). The hydrogels are typically prepared by dissolving the monomers in a proper solvent (e.g. water, buffer solution, methanol) usually in a 50/50 to 10/90 ratio. To form a chemically crosslinked gel, a crosslinker is added, e.g., N,N-methylene bisacrylamide, n-(ethyleneglycol)diacrylate with n = 4-10000, typically in a 1:10 to 1:1000 ratio with respect to the monomer. Further, a photo- or thermal initiator, possibly in combination with an accelerator, is added to initiate the polymerization. The hydrogel layer can be deposited by, e.g., spin coating or by filling a cell with capillary force, as described below. The polymerization of the gel can be induced by temperature or by illumination with UV light. In case of a patterned gel, photo-polymerization is preferred. By tuning the content of the mixture in the appropriate way during polymerization, phase separation can be obtained to form microporous hydrogels (for higher ion permeability and absorbing capacity). Important parameters for this are for example the solvent fraction and the crosslink density. The porosity of the hydrogel might be tuned also by adding polyoxyethylene stearyl ethers (e.g., Brij 700, an emulsion stabilizer). The pore size should be smaller than the cells and is typically in the nm range, e.g., from 5nm - 5$\mu$m and preferably between 10nm - 1 $\mu$m.

The effect of the hydrogel layer on the electric field is very different to that of a layer of insulating material such as $SiO_2$ or SU8. When the electrodes are covered with an insulating layer, there is a large voltage drop over the layer and the field in the volume occupied by the cells is strongly reduced. In order to achieve efficient DEP manipulation of cells, the voltage applied to the electrodes has to be increased correspondingly to compensate for the voltage drop. The electric field becomes then very large at the surface of the insulating layer, which again leads to a high risk for lysis.

In contrast, the hydrogel layer only provides a physical barrier for the cells, but is almost completely permeable to the electric field. This is due to the fact that the layer acts as a sponge and absorbs the surrounding liquid medium. The fraction of liquid in the hydrogel can exceed 90% of its volume in the wet state. The electrical properties of the gel (conductivity and permittivity) are therefore always similar with those of the medium in which the cells are suspended. Consequently, the electric field is only weakly perturbed.

It is also possible to pattern the hydrogel so that only some electrodes are covered with gel and that there are other regions free of hydrogel where intentional E-field lysing or electroporation can be carried out.

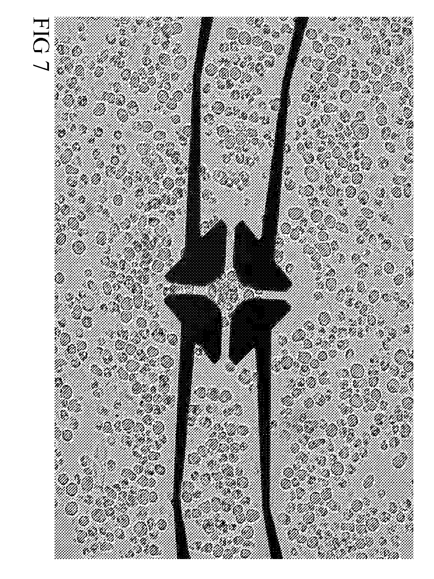

To verify the operation, two identical DEP devices with quadrupolar traps of various sizes were manufactured. The electrodes (Ti/Pt) were deposited by photolithography. In one device, shown in plan view in figure 6, the electrodes were bare and in direct contact with the cell suspension, as in conventional DEP devices. In the second device, shown in figure 7, the electrodes were covered with a hydrogel layer.

For this experiment, the hydrogel solution was obtained by mixing 80% demineralized water and 20% acryl amide / N,N-methylene bisacrylamide (in a 5:1 ratio) and adding 2wt% of Irgacure 2959 (photo-initiator). The substrate was first cleaned with UV ozone and coated with the adhesion promoter 3-Methacryloxypropyltrimethoxysilane (A174). A second (temporary) substrate was coated with a 1H,1H,2H,2H-perfluorodecyltrichlorosilane. Spacer lines of 50 $\mu$m thickness were placed on the edge of 3-Methacryloxypropyltrimethoxysilane -coated substrate and a cell was formed by placing the second substrate on the spacers. The cell was then filled by capillary force with the monomer mixture. After exposing the cell for 20 min to UV light from a Philips PL 10 lamp (intensity 2.6 mW/cm$^2$), a solid hydrogel layer was formed. The top substrate was removed and the hydrogel layer was washed in demineralized water to make sure that residual free monomers and solvent were removed.

Lymphocytes (from the mouse lymphoma EL4 cell line) were used in the experiments. The cells were suspended in a sucrose buffer (290 mM Sucrose, 10 mM HEPES, pH 7.4). The conductivity of the suspension was adjusted to around 400 $\mu$S/cm by adding PBS. Prior to the experiment the devices were filled with a blocking buffer (2% BSA) for 30 minutes to prevent sticking of the cells.

In the device with bare electrodes, the cells showed a strong DEP response at 1V (peak-peak), both for the negative (50 kHz) and positive (500 kHz) DEP regime. Cell lysis was seen to occur when the voltage was increased to between 2 and 3 V, especially in the positive DEP regime (where the cells are drawn towards the electrodes). On the contrary, cell lysis was not seen to occur in the hydrogel device until the voltage was increased 20-30 V (the exact value depending on the proximity of the cells to the electrodes and the DEP regime, with cells lysing predominantly in the pDEP regime). The DEP response of the cells in the hydrogel-coated device was also weaker than with the uncoated device. This was expected as the cells are further away from the electrodes and that the field decreases exponentially in the z-direction (see Fig. 4). The strength of the DEP response can be increased by reducing the thickness of the hydrogel layer.

The next question was to verify that the reduced occurrence of lysis was not simply due to a strong reduction of the electric field in the device, as is the case for layers of insulating materials such as SU8. The strength of the electric field in the two DEP devices was compared by measuring the levitation height of 20 $\mu$m polystyrene beads in water. In this experiment the bead is essentially used as an E-field probe. The conductivity of the suspension was kept to low values (50 $\mu$S/cm) to reduce thermal effects in the sample. A single bead was trapped in the center of the quadrupole device by negative DEP. The voltage was then increased until the bead started to levitate. The levitation height was measured by adjusting the fine focusing wheel on the microscope to keep the bead in focus.

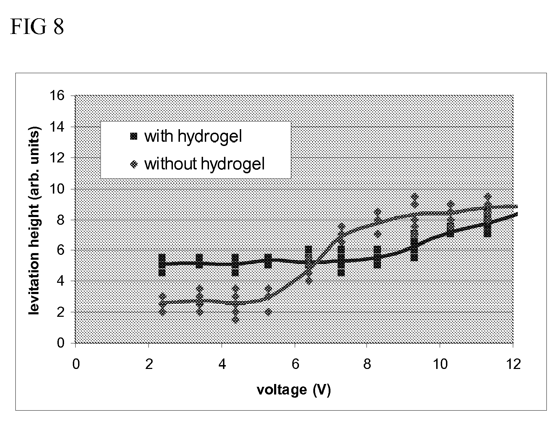

As can be seen in the graph of Fig. 8, a slightly increased voltage has to be applied to the hydrogel-coated sample in order to obtain the same levitation height. Nevertheless, the difference in voltage is only approximately 2-3V, i.e., much less than the difference observed in the lysis experiment. Note that the different offset of the lines (at low voltages) is due to the thickness of the hydrogel layer.

In operation, measuring the crossover frequency proceeds as follows:

1. The cell(s) under investigation are trapped at the center of the DEP cage by means of negative dielectrophoresis. For this purpose an AC field is applied with a low frequency (e.g. 10 to 100 kHz). The voltage level can be in the order of 1 Volt but can be increased or decreased until a sufficient trapping force is generated.

2. The frequency of the AC voltage is now gradually increased (e.g. beyond 100 kHz) until a movement of the cell (s) away from the center of the cage, towards the electrodes, is observed. The frequency at which this occurs, is the crossover frequency.

3. This process can now be repeated for different experimental conditions. For instance a chemical agent that permeabilizes the cell to a certain (known or unknown) extent can be added to the medium. Or an electropulse can be applied, either using the already present electrodes, or using additional electrodes. A simple way of doing this, using the existing quadrupole electrodes, is to briefly attract the cell(s) toward a higher field region in the cage by positive DEP using a high frequency (> 1 MHz). The cell can be held at a certain pre-determined position in the cage by rapidly switching between low and high frequency fields using the cell presence sensing device such as a photodiode for position feedback. Hereafter, a possible change in crossover frequency can be detected by repeating steps 1 and 2.

An explanation follows of dielectrophoresis in quadrupole traps based on the so-called effective moment method as described by Jones T.B., "Basic theory of dielectrophoresis and electrorotation", IEEE Engineering in Medicine and Biology Magazine, November/December 2003, pp 33-42.

The DEP force on a particle can be expressed as the sum of the gradients of a set of electromechanical potentials $U_n$, where n = 1, 2, 3, etc., corresponding respectively to the dipole, quadrupolar, octopolar etc. components of the induced multipole potential:

$$F = -\nabla\left(U_1 + U_2 + \cdots\right).$$

In the remainder we will only consider the first two terms (dipole and quadrupole) as the subsequent terms contribute only very little to the resulting force. The quadrupolar term, however, is of significant importance in the quadrupole electrode configuration. For instance, it is this term that causes levitation of particles along the centreline of the trap. The terms $U_1$ and $U_2$ are given by Jones T.B., "Basic theory of dielectrophoresis and electrorotation", IEEE Engineering in Medicine and Biology Magazine, November/December 2003, pp 33-42:

$$U_1 = -\pi R^3 \varepsilon_1 \operatorname{Re}(K_1)\left[\left(\frac{\partial \Phi}{\partial x}\right)^2 + \left(\frac{\partial \Phi}{\partial y}\right)^2 + \left(\frac{\partial \Phi}{\partial z}\right)^2\right],$$

$$U_2 = -\frac{2}{3}\pi R^5 \varepsilon_1 \operatorname{Re}(K_2)\left\{\frac{1}{2}\left[\left(\frac{\partial^2 \Phi}{\partial x^2}\right)^2 + \left(\frac{\partial^2 \Phi}{\partial y^2}\right)^2 + \left(\frac{\partial^2 \Phi}{\partial z^2}\right)^2\right] + \left(\frac{\partial^2 \Phi}{\partial y \partial z}\right)^2 + \left(\frac{\partial^2 \Phi}{\partial z \partial x}\right)^2 + \left(\frac{\partial^2 \Phi}{\partial x \partial y}\right)^2\right\}$$

Here $\Phi$ is the electric potential (more precisely: the RMS value of the AC potential), R the radius of the (assumedly spherical) particle and $\varepsilon_1$ the permittivity of the suspending medium. The terms $K_1$ and $K_2$ are the so-called Clausius-Mossotti factors. These are complex, frequency dependent terms, and they determine the sign of the dielectrophoretic force on the particles under study. These terms depend on the size of the particle, and on the electric properties (permittivity and conductivity) of the (constituents of the) particle and the surrounding medium. For example in Figure 9 a graph is shown of a typical variation of $\operatorname{Re}(K_1)$ and $\operatorname{Re}(K_2)$ with frequency for the case where the cell is modelled as a single-shell particle, consisting of a non-conductive membrane encapsulating a conductive cytoplasm.

It can be observed from Figure 9 that the crossover frequencies of $\operatorname{Re}(K_1)$ and $\operatorname{Re}(K_2)$ (i.e. the frequencies where the graphs intersect the horizontal axis) are different. This fact influences the general crossover behaviour of cells in the trap, i.e., the transition from negative DEP (nDEP) to positive DEP (pDEP) or vice versa. If the quadrupolar term were absent, the crossover behavior would be binary. That is, for all locations in the trap, there would either be nDEP or pDEP, depending on the frequency. In this case, the cell would either move to the center of the trap (nDEP) or to the position with the highest electric field (at or near the electrodes). However, because the crossover frequencies of RE $(K_1)$ and $\operatorname{Re}(K_2)$ are different, it will occur that in a narrow frequency band (for example in a band of about 5 to 10 kHz) between the crossover frequencies, the dipolar and quadrupolar terms oppose each other. This will result in the occurrence of stable equilibrium positions in the region between the electrode edges and the center of the trap. Therefore, instead of binary, the transition between nDEP and pDEP can be expected to be more gradual. Hence the exact position of a cell or cells within the cage is controllable. The exact location of the equilibrium points depends on the electric potential distribution in the trap, the applied frequency and the (di)electric properties of the particle and the medium under study.

To quantify this effect, it is convenient to model the field in the quadrupole trap using the parametric model for azimuthally periodic electrodes proposed by Jones T.B., "Basic theory of dielectrophoresis and electrorotation", IEEE Engineering in Medicine and Biology Magazine, November/December 2003, pp 33-42. In this approximation the potential is given as:

$$\Phi = V\left\{a + bz + c\left[6z^2 - \rho^2\right] + d\left[6z^3 - 3z\rho^2\right]\right\}\rho^2 \cos 2\theta ,$$

where the position in the trap is given in cylindrical coordinates ($\rho$, $\theta$, z): (radius, azimuth, height).

For a given electrode configuration, and given electric properties of the medium, the parameters *a, b,* c, and d can be estimated or calculated. Subsequently, the electromechanical potentials $U_1$ and $U_2$, and the associated DEP force terms can be computed. The equilibrium points are given by the local minima in the total electromechanical potential U = $U_1$+$U_2$.

The effect of the opposing dipolar and quadrupolar force terms can be used as a refined way to visualize and/or measure the effect of the permeability state of the cell membrane. A change in the porosity of the membrane will change the factors Re($K_1$) and Re($K_2$) and cause a detectable/measurable shift of the DEP equilibrium position. This shift can be less than the complete shift from the center of the cage to an electrode, hence a finer determination of a change in permeability may be detected.

In the embodiment described above the state of a cell membrane can be determined by detecting a change in the crossover frequency. The basic method consists of a procedure in which the applied frequency is varied, and a cell presence sensor such as an optical sensor (e.g. an integrated PIN diode) as part of a cell movement detection system according to embodiments of the present invention is used to detect movement of the cell. In this way it can be determined whether the cell is undergoing nDEP or pDEP. The frequency at which the transition from nDEP to pDEP occurs is the crossover frequency. However, the analysis involving the quadrupolar term, as given in the previous section, implies allows this method can be refined. Basically, the precise movement of the particle at frequencies close to the crossover range can be predicted on the basis of the model given above. Deviations from the predicted behavior can be related to changes in the electric properties of the cell under study, which in turn are due to changes in the integrity of the cell membrane. To achieve the small changes in frequency at the crossover point, the AC field generator may be adapted to a have a fine frequency control that allows small changes of frequency when investigating the characteristics of cells concerned.

For example, the equilibrium points of the cell position in the frequency range at which the transition from nDEP to pDEP occurs can be related to specific (di)electric properties of the cell. For a given, fixed, frequency, a displacement in the equilibrium position of the cell indicates a change in the integrity of the cell membrane.

The barrier layer can be applied anywhere the electric field is higher, e.g. to electrodes of any devices equipped with microelectrodes for electric field manipulation of cells or in channel restrictions. These include devices for transporting cells, focusing them or trap them in field cages. Although the embodiments described are focused on dielectrophoresis, the invention could also be applied to microelectrode devices that additionally use other (fluid) pump mechanisms to transport cells (e.g., electrothermal effects).

**Claims**

1. A device for applying an electric field to particles in a fluid, the device having electrodes arranged to generate the electric field, and drive circuitry to drive the electrodes at selectable AC frequencies, the device also having a barrier layer to keep the particles in the fluid away from the areas of higher electric field intensity, the barrier layer being permeable to the electric field.

2. The device of claim 1, being arranged for dielectrophoresis.

3. The device of claim 2, wherein the barrier layer is made of a material and has a sufficient thickness so that the field strength in the fluid is reduced so that the risk of lysis of a cell is reduced.

4. The device of claim 2, wherein the barrier layer is made of a material and has a sufficient thickness so that that a change from negative to positive DEP, or vice versa, an be detected as an observable change of position of the cell.

5. The device of any previous claim, the barrier layer being formed of a porous or non-porous hydrogel material, or a porous membrane.

6. The device of any preceding claim, the particles comprising cells, and the barrier layer being of such a thickness, and the drive circuitry being arranged such that the field strength in the fluid adjacent to the barrier layer is sufficiently low to avoid cell lysis.

7. The device of any preceding claim wherein the barrier layer is applied to some parts of the electrode and not to others.

8. The device of any preceding claim, the barrier being thicker at parts of the electrode surface subject to higher electrical field strength.

9. The device of claim 2 or any claim when dependent on claim 2, being arranged to detect a crossover frequency for at least one of the particles, the drive circuitry being arranged to alter the AC frequency, so that the net force on the particle changes direction between the directions of increasing field strength and decreasing field strength and the device being arranged to detect such a change.

10. The device of any preceding claim, the electrodes and the drive circuitry being arranged so as to provide an electric field in the fluid in the form of a trough or peak, to retain the particles which have a net force in a direction of decreasing or increasing field strength respectively.

11. An integrated micro fluidic device having integrated fluid handling components, and having the device of any preceding claim.

12. A method of manipulating cells in a fluid using a dielectrophoretic device, the device having electrodes arranged to generate the electric field, and circuitry to drive the electrodes at selectable AC frequencies, the electrodes having a barrier layer on a surface of the electrodes facing the fluid, to keep the cells in the fluid away from the surface of the electrodes, the method having the steps of causing cells to enter the device, and controlling the AC frequency to cause the cells to move in a desired direction in the fluid.

13. The method of claim 12 having the step of measuring a frequency at which crossover occurs.

14. A method of manufacturing a dielectrophoresis device for applying an electric field to particles in a fluid, the method having the steps of forming electrodes arranged to generate the electric field, forming drive circuitry to drive the electrodes at selectable AC frequencies, and forming a barrier layer on a surface of the electrodes facing the fluid, to keep the particles in the fluid away from the surface of the electrodes.

FIG 1

FIG 2a

FIG 2b

FIG 2c

FIG 3

FIG 4

FIG 5

FIG 6

FIG 7

FIG 8

FIG 9

**European Patent Office**

## EUROPEAN SEARCH REPORT

Application Number

EP 07 11 9255

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/146100 A1 (HUANG YING [US] ET AL HUANG YING [US] ET AL) 7 August 2003 (2003-08-07) * paragraphs [0060], [0061] * ----- | 1-14 | INV. B03C5/02 |
| X | WO 2007/107947 A (KONINKL PHILIPS ELECTRONICS NV [NL]; PONJEE MARC W G [NL]; GILLIES MUR) 27 September 2007 (2007-09-27) * page 9, line 1 - line 3 * ----- | 1-14 | |
| X | US 2002/155586 A1 (CHENG JING [CN] ET AL CHENG JING [CN] ET AL) 24 October 2002 (2002-10-24) * paragraph [0060] * ----- | 1-14 | |
| | | | TECHNICAL FIELDS SEARCHED (IPC) B03C |

The present search report has been drawn up for all claims

| Place of search | Date of completion of the search | Examiner |
|---|---|---|
| The Hague | 8 July 2008 | Demol, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 11 9255

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2008

| Patent document cited in search report | Publication date | Patent family member(s) | Publication date |
|---|---|---|---|
| US 2003146100 A1 | 07-08-2003 | NONE | |
| WO 2007107947 A | 27-09-2007 | WO 2007107892 A1<br>WO 2007107934 A1 | 27-09-2007<br>27-09-2007 |
| US 2002155586 A1 | 24-10-2002 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007010367 A

- US 6838053 B

**Non-patent literature cited in the description**

- **HUANG, Y. ; RUBINSKY, B.** Microfabricated electroporation chip for single cell membrane permeabilization. *Sensors and Actuators A,* 2001, vol. 89, 242-249
- **HUANG, Y. et al.** Instantaneous, quantitative single-cell viability assessment by electrical evaluation of cell membrane integrity with micro fabricated devices. *Sensors and Actuators A,* 2003, vol. 105, 31-39

- **JONES T.B.** Basic theory of dielectrophoresis and electrorotation. *IEEE Engineering in Medicine and Biology Magazine,* November 2003, 33-42